(19) Europäisches Patentamt
European Patent Office
Office européen des brevets

(11) **EP 1 955 652 A1**

(12) **EUROPEAN PATENT APPLICATION**
published in accordance with Art. 158(3) EPC

(43) Date of publication:
**13.08.2008 Bulletin 2008/33**

(51) Int Cl.:
***A61B 5/1455*** (2006.01)

(21) Application number: **06812001.3**

(22) Date of filing: **19.10.2006**

(86) International application number:
**PCT/JP2006/320813**

(87) International publication number:
**WO 2007/046455 (26.04.2007 Gazette 2007/17)**

(84) Designated Contracting States:
**DE**

(30) Priority: **21.10.2005 JP 2005306902**

(71) Applicant: **MATSUSHITA ELECTRIC INDUSTRIAL CO., LTD.**
**Osaka 571-8501 (JP)**

(72) Inventors:
• **SHIOI, Masahiko,**
**Matsushita Elec. Ind. Co., Ltd.,**
**Intellectual Prop. Right Operations Co.**
**Osaka 540-6207 (JP)**

• **MIYAMOTO, Yoshiko,**
**Matsushita Elec. Ind. Co., Ltd.,**
**Intellectual Prop. Right Operations Co.**
**Osaka 540-6207 (JP)**
• **UCHIDA, Shinji,**
**Matsushita Elec. Ind. Co., Ltd.,**
**Intellectual Prop. Right Operations Co.**
**Osaka 540-6207 (JP)**

(74) Representative: **TBK-Patent**
**Bavariaring 4-6**
**80336 München (DE)**

(54) **BIOMETRIC INFORMATION MEASURING DEVICE**

(57) A bioinformation measurement device that enables further accurate bioinformation measurement is provided. The device includes an insertion portion 104 to be inserted in an ear cavity 200; a first light inlet 105 and a second light inlet 106 provided at the insertion portion 104, for introducing the infrared light irradiated from the ear cavity 200 to the insertion portion 104; an optical guide path provided in the insertion portion 104 for guiding the first infrared light introduced from the first light inlet 105 and the second infrared light introduced from the second light inlet 106; a dispersive element for dispersing the first infrared light and the second infrared light guided by the optical guide path; an infrared ray detector 108 for detecting the first infrared light and the second infrared light dispersed by the dispersive element; and a computing unit for computing bioinformation based on the intensities of the first infrared light and the second infrared light detected by the infrared ray detector 108.

FIG. 2

**Description**

Technical Field

The present invention relates to a bioinformation measurement device which noninvasively measures bioinformation by using infrared irradiated light from the ear cavity.

Background Art

As a conventional bioinformation measurement device, there has been proposed a device that noninvasively measures a living subject, particularly a blood-sugar level by using infrared irradiated light from the eardrum (for example, patent document 1). For example, patent document 1 discloses a device that determines a blood-sugar level with an infrared ray detector by noninvasively measuring a radiation naturally generated from eardrums as heat in the infrared range of the spectrum, and having a spectrum that is distinctive of human organs.
Patent Document 1 Japanese Unexamined Patent Application No. 2001-503999

Disclosure of the Invention

Problem to be Solved by the Invention

According to Planck's law, however, any object having a temperature inevitably emits an infrared radiation due to the heat. In the case of the above conventional measurement device, not only the eardrum, but the external ear canal is also a radiant of infrared light. Thus, irradiated light from the eardrum and irradiated light from the external ear canal enter the infrared ray detector.
The irradiated light from the external ear canal is considered a noise, since the irradiated light from the external ear canal contains less information on blood compared with the irradiated light from the eardrum, because the skin of the external ear canal is thick compared with that of the eardrum and the blood supply is at a relatively deeper position. Thus, the irradiated light from the external ear canal has been a factor of inaccurate measurement.
Considering the above conventional problem, the present invention aims to provide a bioinformation measurement device which can carry out a further accurate bioinformation measurement.

Means for Solving the Problem

To solve the above conventional problem, a bioinformation measurement device of the present invention for measuring bioinformation based on an intensity of infrared light includes:

an insertion portion to be inserted into an ear cavity;
a first light inlet and a second light inlet provided at the insertion portion, for introducing infrared light irradiated from the ear cavity into the insertion portion;
an optical guide path provided in the insertion portion, for guiding first infrared light introduced from the first light inlet and second infrared light introduced from the second light inlet;
a dispersive element for dispersing the first infrared light and the second infrared light guided by the optical guide path; and
an infrared ray detector for detecting the first infrared light and the second infrared light dispersed by the dispersive element.

Effect of the Invention

Based on the bioinformation measurement device of the present invention, a further accurate bioinformation measurement can be carried out by considering the effects of the external ear canal on the measurement.

Brief Description of the Drawings

[FIG. 1] A perspective illustration showing an external view of a bioinformation measurement device in one embodiment of the present invention.
[FIG. 2] A diagram showing a configuration of the bioinformation measurement device.

[FIG. 3] A perspective illustration showing an insertion portion and a shutter of the bioinformation measurement device.

[FIG. 4] A perspective illustration showing an optical filter wheel of the bioinformation measurement device.

[FIG. 5] An illustration showing a configuration of an example of a first variation of the bioinformation measurement device.

[FIG. 6] A perspective illustration showing an insertion portion of the bioinformation measurement device in another embodiment of the present invention.

[FIG. 7] An illustration of a configuration of the bioinformation measurement device.

[FIG. 8] A perspective illustration showing an example of a variation of the insertion portion of the bioinformation measurement device.

Best Mode for Carrying Out the Invention

A bioinformation measurement device of the present invention for measuring bioinformation based on an intensity of infrared light includes:

an insertion portion to be inserted into an ear cavity;

a first light inlet and a second light inlet provided at the insertion portion, for introducing infrared light irradiated from the ear cavity into the insertion portion;

an optical guide path provided in the insertion portion, for guiding first infrared light introduced from the first light inlet and second infrared light introduced from the second light inlet;

a dispersive element for dispersing the first infrared light and the second infrared light guided by the optical guide path; and

an infrared ray detector for detecting the first infrared light and the second infrared light dispersed by the dispersive element. Further preferably, a computing unit for computing bioinformation based on the intensities of the first infrared light and the second infrared light detected by the infrared ray detector is further included.

In the present invention, for the optical guide path, any optical guide path may be used as long as it can introduce infrared light: for example, a hollow pipe, and optical fiber that transmits the infrared light. When the hollow pipe is to be used, a gold layer is preferably provided at the inner surface of the hollow pipe. The gold layer may be formed by gold-plating, or by vapor depositing gold at the inner surface of the hollow pipe.

For the dispersive element, any dispersive element may be used as long as it can disperse infrared light by wavelength: for example, an optical filter, a spectroscopic prism, a Michelson interferometer, and a diffraction grating, which transmit infrared light in a specific wavelength range.

For the infrared ray detector, any infrared ray detector may be used as long as it can detect the light with a wavelength in the infrared range: for example, a pyroelectric sensor, a thermopile, a bolometer, a HgCdTe (MCT) detector, and a Golay cell.

A plurality of the infrared ray detectors may be provided.

For the computing unit, for example, a microcomputer such as CPU (Central Processing Unit) may be used.

The bioinformation measurement device of the present invention may include a plurality of optical guide paths, including a first optical guide path for guiding the first infrared light introduced from the first light inlet, and a second optical guide path for guiding the second infrared light introduced from the second light inlet.

The first infrared light and the second infrared light may also be guided by one optical guide path.

In the bioinformation measurement device of the present invention, the second light inlet is preferably configured so that the infrared light irradiated from the eardrum is not introduced.

With such a configuration, since the infrared light irradiated from the eardrum is not introduced from the second light inlet, the second infrared light introduced from the second light inlet corresponds only to the infrared light irradiated from the external ear canal. Thus, by using the intensity of the first infrared light including the infrared light irradiated from the eardrum and the infrared light irradiated from the external ear canal, and the intensity of the second infrared light, and correcting the effects of the infrared light irradiated from the external ear canal, a further accurate bioinformation measurement based on the infrared light irradiated from the eardrum can be carried out.

In the bioinformation measurement device of the present invention, the insertion portion may include an end portion that is directed toward the eardrum when inserted into the ear cavity; and a side face. The first light inlet may be provided at the end portion of the insertion portion. Further, the second light inlet is preferably provided at the side face of the insertion portion.

The bioinformation measurement device of the present invention preferably further includes a shielding portion provided at the insertion portion, for shielding the second light inlet from the infrared light irradiated from the eardrum. With such a configuration, the infrared light irradiated from the eardrum is not introduced from the second light inlet, and therefore the second infrared light introduced from the second light inlet corresponds only to the infrared light irradiated from the external ear canal. Thus, by using the intensity of the first infrared light including the infrared light irradiated from the eardrum and the infrared light irradiated from the external ear canal, and the intensity of the second infrared light, and correcting the effects of the infrared light irradiated from the external ear canal on the measurement, a further accurate bioinformation measurement based on the infrared light irradiated from the eardrum can be carried out.

The surface of the shielding portion is preferably formed of gold, silver, copper, brass, aluminum, platinum, or iron; and the surface of the shielding portion is preferably glossy.

Preferably, the shielding portion is provided removably at the insertion portion.

The bioinformation measurement device of the present invention may further include an optical path control unit for controlling the optical path of the infrared light reaching the infrared ray detector. The optical path control unit is preferably able to control the optical path so that the infrared light reaching the infrared ray detector can be switched between the first infrared light and the second infrared light, and the first infrared light only.

For the optical path control unit, a shutter, and an aperture may be mentioned.

In the bioinformation measurement device of the present invention, the computing unit may further include a warning output unit for making comparison between the threshold and the intensity difference between the first infrared light intensity and the second infrared light intensity, and outputting a warning when the intensity difference is larger than the threshold. With such a configuration, a user can be notified of an inappropriate position of the bioinformation measurement device.

For the warning output unit, a display for showing the warning, a speaker for outputting a warning with a sound, and a buzzer for producing a warning sound may be mentioned.

The bioinformation measurement device of the present invention further may include a memory unit for storing correlational data showing the correlation between the output signal of the infrared ray detector and the bioinformation; a display unit for displaying bioinformation converted by the computing unit; and a power source for supplying electrical power for the bioinformation measurement device to be in operation.

The computing unit may convert the output signal of the infrared ray detector to bioinformation, by reading the above correlational data from the memory unit and referring to it.

The correlational data showing the correlation between the output signal of the infrared ray detector and bioinformation can be obtained, for example, by measuring the output signal of the infrared ray detector on a patient with known bioinformation (for example, a blood-sugar level), and analyzing the obtained correlation between the output signal of the infrared ray detector and the bioinformation.

In the present invention, for the memory unit, for example, a memory such as RAM and ROM may be used.

For the display unit, for example, a display of liquid crystal may be used.

For the power source, for example, a battery may be used.

For the bioinformation as the measurement target of the present invention, a glucose concentration (a blood-sugar level), a hemoglobin concentration, a cholesterol concentration, a neutral fat concentration, and a protein concentration may be mentioned.

By measuring the infrared light irradiated from a living subject, bioinformation, for example, a blood-sugar level can be measured. Radiant power W of the infrared irradiated light from the living subject is represented by the mathematical expression below.

[Mathematical Expression 1]

$$W = s \int_{\lambda_1}^{\lambda_2} \varepsilon(\lambda) \cdot W_0(T, \lambda) d\lambda \, (W)$$

[Mathematical Expression 2]

$$W_0(\lambda,T) = 2hc^2 \left\{ \lambda^5 \cdot \left[ \exp(hc/\lambda kT) - 1 \right] \right\}^{-1} \left( W / cm^2 \cdot \mu m \right)$$

The respective symbols in the above expressions represent the following.
W: Radiant Power of The Infrared Irradiated Light From Living Subject
$\varepsilon(\lambda)$: Emissivity of Living Subject at Wavelength $\lambda$
$W_0(\lambda,T)$: Spectral Radiant Emittance of Blackbody at wavelength $\lambda$, and temperature T
h: Plank's constant (h=6.625 $\times$ 10$^{-34}$ (W·S$^2$))
c: Light Velocity (c=2.998 $\times$ 10$^{10}$(cm/s))
$\lambda_1$, $\lambda_2$: Wavelength ($\mu$m) of Infrared Irradiated Light from Living Subject
T: Temperature (K) of Living Subject
S: Detected Area (cm$^2$)
k: Boltzmann constant
As is clear from Mathematical Expression 1, when detected area S is constant, radiant power W of the infrared irradiated light from a living subject depends on emissivity $\varepsilon(\lambda)$ of the living subject at wavelength $\lambda$. Based on Kirchhoff's law of radiation, emissivity equals absorptivity at the same temperature and the same wavelength.
[Mathematical Expression 3]

$$\varepsilon(\lambda) = \alpha(\lambda)$$

In Mathematical Expression 3, $\alpha(\lambda)$ represents the absorptivity of a living subject at wavelength $\lambda$. Therefore, upon considering the emissivity, the absorptivity may be considered. Based on the law of conservation of energy, absorptivity, transmittance, and reflectivity satisfy the following relation.
[Mathematical Expression 4]

$$\alpha(\lambda) + r(\lambda) + t(\lambda) = 1$$

The respective symbols in the above expression represent the following.
$r(\lambda)$: Reflectivity of Living Subject at wavelength $\lambda$
$t(\lambda)$: Transmittance of Living Subject at wavelength $\lambda$
Therefore, the emissivity can be expressed as, by using the transmittance and the reflectivity:
[Mathematical Expression 5]

$$\varepsilon(\lambda) = \alpha(\lambda) = 1 - r(\lambda) - t(\lambda)$$

The transmittance is expressed as the ratio of the amount of incident light to the amount of the transmitted light that was transmitted through the measurement subject. The amount of incident light and the amount of the transmitted light upon being transmitted through the measurement subject are shown with Lambert-Beer law.
[Mathematical Expression 6]

$$I_t(\lambda) = I_0(\lambda) \exp\left(-\frac{4\pi k(\lambda)}{\lambda} d\right)$$

The respective symbols in the above expression represent the following.
It: Amount of the Transmitted Light
$I_0$: Amount of Incident Light
d: Thickness of Living Subject
$k(\lambda)$: Extinction Coefficient of living subject at Wavelength $\lambda$.
The extinction coefficient of living subject is a coefficient showing the light absorption by living subject.
Therefore, the transmittance can be expressed as:
[Mathematical Expression 7]

$$t(\lambda) = \exp\left(-\frac{4\pi k(\lambda)}{\lambda} d\right)$$

The reflectivity is described next. Regarding reflectivity, an average of the reflectivities of all directions has to be calculated. However, for simplification, the reflectivity in normal incidence is considered. The reflectivity in normal incidence is expressed as the following, setting the refractive index of air as 1:
[Mathematical Expression 8]

$$r(\lambda) = \frac{(n(\lambda)-1)^2 + k^2(\lambda)}{(n(\lambda)+1)^2 + k^2(\lambda)}$$

In the expression, $n(\lambda)$ shows the refractive index of living subject at wavelength $\lambda$.
From the above, the emissivity is expressed as:
[Mathematical Expression 9]

$$\varepsilon(\lambda) = 1 - r(\lambda) - t(\lambda) = 1 - \frac{(n(\lambda)-1)^2 + k(\lambda)^2}{(n(\lambda)+1)^2 + k(\lambda)^2} - \exp\left(-\frac{4\pi k(\lambda)}{\lambda}d\right)$$

When the concentration of a component in a living subject changes, the refractive index and the extinction coefficient of the living subject change. The reflectivity is low, usually about 0.03 in the infrared range, and as can be seen from Mathematical Expression 8, it is not much dependent on the refractive index and the extinction coefficient. Therefore, even the refractive index and the extinction coefficient change with changes in the concentration of a component in living subject, the changes in the reflectivity is small.

On the other hand, the transmittance depends, as is clear from Mathematical Expression 7, heavily on the extinction coefficient. Therefore, when the extinction coefficient of a living subject, i.e., the degree of light absorption by the living subject, changes by changes in the concentration of a component in a living subject, the transmittance changes.

The above clarifies that the radiant power of infrared irradiated light from a living subject depends on the concentration of a component in the living subject. Therefore, a concentration of a component in a living subject can be determined from the radiant power intensity of the infrared irradiated light from the living subject.

Also, as is clear from Mathematical Expression 7, the transmittance is dependent on the thickness of a living subject. The smaller the thickness of the living subject, the larger the degree of the change in the transmittance relative to the change in the extinction coefficient of the living subject, and therefore changes in the component concentration in a living subject can be easily detected. Since eardrums have a small thickness of about 60 to 100 $\mu$m, it is suitable for a concentration measurement of a component in a living subject using infrared irradiated light.

In the following, embodiments of the present invention are described by referring to the figures.

Embodiment 1

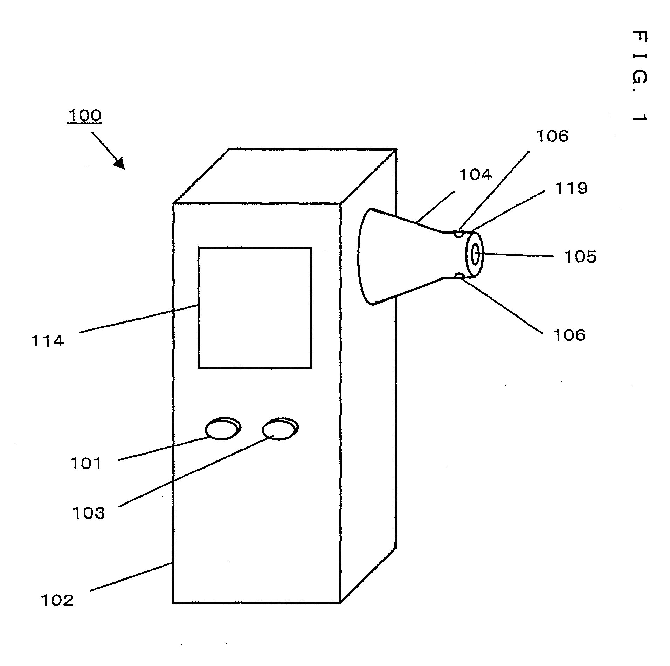

FIG. 1 is a perspective illustration showing an external view of a bioinformation measurement device 100 of Embodiment 1.

The bioinformation measurement device 100 includes a main body 102, and an insertion portion 104 provided on the side face of the main body 102. The main body 102 includes a display 114 for displaying the measurement results of the concentration of a component in a living subject, a power source switch 101 for ON/OFF of a power source of the bioinformation measurement device 100, and a measurement start switch 103 for starting the measurement. At the insertion portion, a first light inlet 105 for introducing infrared light irradiated from an ear cavity into the bioinformation measurement device 100, and two second light inlets 106 are provided.

The first light inlet 105 is provided at an end (end portion) of the insertion portion 104, and is directed toward the eardrum upon the insertion portion 104 is inserted into the ear cavity. The two second light inlets 106 are provided on the side faces of the insertion portion 104.

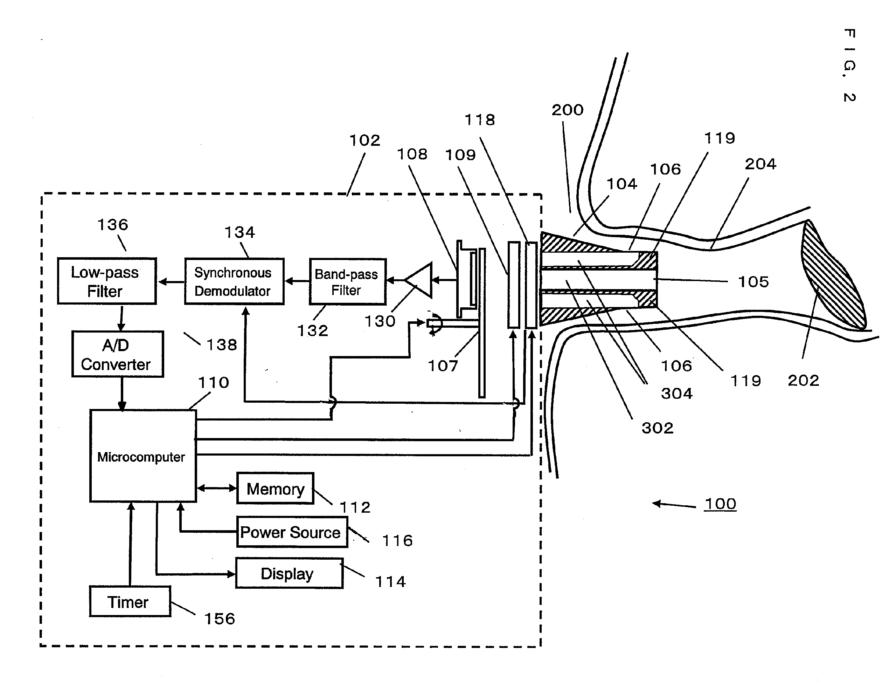

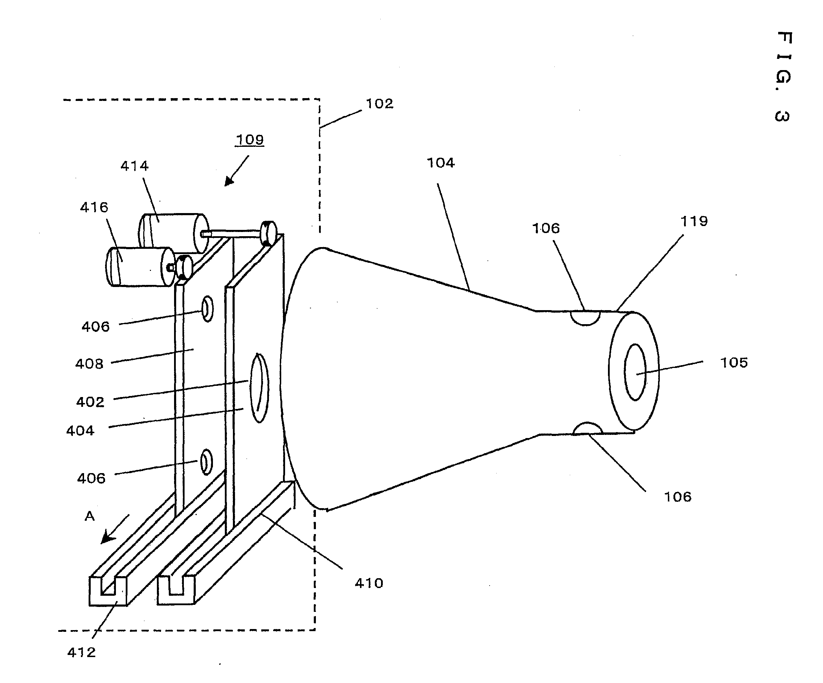

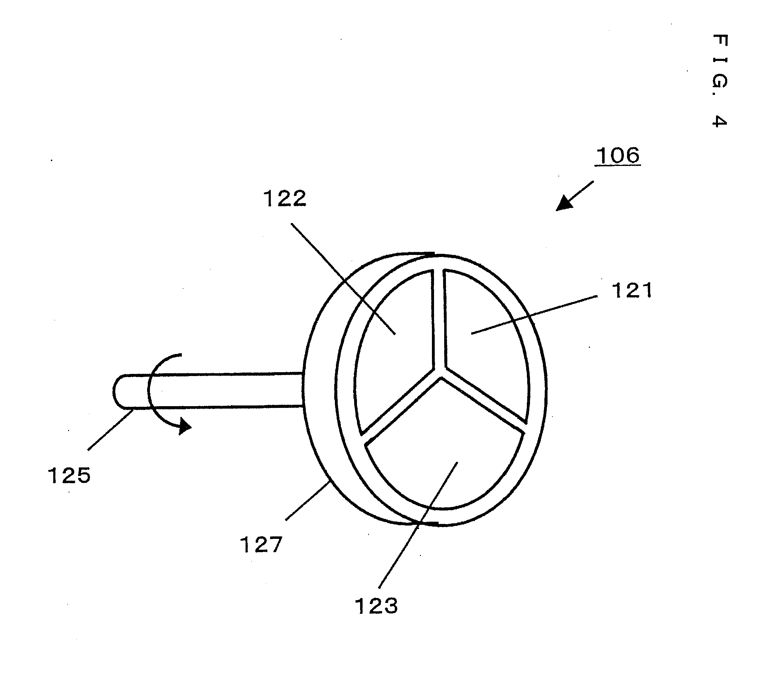

Next, an internal structure of the main body of the bioinformation measurement device 100 is described by using FIG. 2, FIG. 3, and FIG. 4. FIG. 2 is a diagram showing a configuration of a bioinformation measurement device 100 of Embodiment 1; FIG. 3 is a perspective illustration showing the insertion portion 104 and a shutter 109 of the bioinformation measurement device 100 of Embodiment 1; and FIG. 4 is a perspective illustration of an optical filter wheel 107 of the bioinformation measurement device 100 of Embodiment 1. In FIG. 3, a chopper is omitted.

Inside the main body of the bioinformation measurement device 100, a chopper 118, a shutter 109, an optical filter wheel 107, an infrared ray detector 108, a preliminary amplifier 130, a band-pass filter 132, a synchronous demodulator 134, a low-pass filter 136, an analog/digital converter (hereinafter abbreviated as A/D converter) 138, a microcomputer 110, a memory 112, a display 114, a power source 116, a timer 156, and a buzzer 158 are included. In this arrangement, the microcomputer 110 corresponds to the computing unit of the present invention.

The power source 116 supplies an alternating current (AC) or a direct current (DC) to the microcomputer 110. For the power source 116, batteries are preferably used.

The chopper 118 has functions of chopping the light irradiated from the eardrum 202, i.e., the first infrared light introduced into the main body 102 through a first optical guide path 302 provided in the insertion portion 104 from the first light inlet 105 and the second infrared light introduced into the main body 102 through a second optical guide path 304 provided in the insertion portion 104 from the second light inlet 106; and converting the first and the second infrared light into high-frequency infrared ray signals. The operation of the chopper 118 is controlled based on control signals from the microcomputer 110.

The infrared light chopped by the chopper 118 reaches the shutter 109. The shutter 109 has functions of controlling the optical path of the infrared light introduced into the main body 102. The shutter 109 includes, as shown in FIG. 3, a first shield plate 404 with a first aperture 402 corresponding to the optical guide path 302, a second shield plate 408 with two second apertures 406 corresponding to the second optical guide paths 304, a first motor 414 for driving the first shield plate 404 to slide along a first guide 410, and a second motor 416 for driving the second shield plate 408 to slide along a second guide 412.

By driving the second motor 416 and sliding the second shield plate 408 along with the second guide 412 from the position shown in FIG. 3 in the direction of arrow A, the first infrared light introduced by the first optical guide path 302 is blocked by the second shield plate 408, and only the second infrared light introduced by the second optical guide paths 304 reaches the optical filter wheel 107 through the second apertures 406.

On the other hand, by driving the first motor 414 and sliding the first shield plate 404 along with the first guide 410 from the position shown in FIG. 3 in the direction of arrow A, the second infrared light introduced by the second optical guide paths 304 is blocked by the first shield plate 404, and only the first infrared light introduced by the first optical guide path 302 reaches the optical filter wheel 107 through the first aperture 402. With such an arrangement, the infrared light reaching the optical filter wheel 107 can be switched between the first infrared light and the second infrared light. The operation of the shutter 109 is controlled based on the control signal from the microcomputer 110. The shutter 109 corresponds to the optical path control unit of the present invention.

In the optical filter wheel 107, as shown in FIG. 4, a first optical filter 121, a second optical filter 122, and a third optical filter 123 are put in a ring 127. In an example shown in FIG. 4, a disk-like member is formed by putting the first optical filter 121, the second optical filter 122, and the third optical filter 123, all of which are fan-shaped, in the ring 127, and a shaft 125 is provided at the center of the disk-like member.

By rotating this shaft 125 following the arrow in FIG. 4, the optical filter for the infrared light chopped by the chopper 118 to passes through can be switched between the first optical filter 121, the second optical filter 122, and the third optical filter 123. The rotation of the shaft 125 is controlled by the control signal from the microcomputer 110. The optical filter wheel 107 corresponds to the dispersive element of the present invention.

The optical filter may be made by any known methods without particular limitation. For example, vapor deposition methods may be used. The optical filter may be made by the vacuum deposition method, making a layer of for example $ZnS$, $MgF_2$, and $PbTe$ on a base plate using $Si$ or $Ge$.

The infrared light transmitted through the first optical filter 121, the second optical filter 122, and the third optical filter 123 reaches the infrared ray detector 108 including a detection region 126. The infrared light that reached the infrared ray detector 108 enters the detection region 126, and is converted to an electric signal corresponding to the intensity of the infrared light entered.

The rotation of the shaft 125 of the optical filter wheel 107 is preferably synchronized with the operation of the chopper 118, and controlled so that the shaft 125 is rotated to 120 degrees while the chopper 118 is closed. With such an arrangement, when the chopper 118 is opened next time, the optical filter for the infrared light chopped by the chopper 118 to pass through can be switched to the next optical filter.

Also, the operation of the shutter 109 is preferably synchronized with the rotation of the shaft 125, and the operation of the shutter 109 is controlled so that the infrared light passing through the shutter 109 is switched between the first infrared light and the second infrared light every three operations of the shaft 125 to a revolution of 360 degrees.

By controlling the rotation of the shaft 125 and the operation of the shutter 109 in such a manner, the infrared light reaching the infrared ray detector 108 can be switched in the following order: the first infrared light that is transmitted through the first optical filter 121, the first infrared light that is transmitted through the second optical filter 122, the first infrared light that is transmitted through the third optical filter 123, the second infrared light that is transmitted through the first optical filter 121, the second infrared light that is transmitted through the second optical filter 122, and the second infrared light that is transmitted through the third optical filter 123.

The electric signal outputted from the infrared ray detector 108 is amplified by the preliminary amplifier 130. In the amplified electric signal, the signal outside the center frequency, i.e., the frequency band of the chopping, is removed by the band-pass filter 132. Based on this, noise caused by statistical fluctuation such as thermal noise can be minimized.

The electric signal filtered by the band-pass filter 132 is demodulated to DC signal by the synchronous demodulator 134, by synchronizing and integrating the chopping frequency of the chopper 118 and the electric signal filtered by the band-pass filter 132.

In the electric signal demodulated by the synchronous demodulator 134, the signal in the high-frequency band is removed by the low-pass filter 136. Based on this, noise is further removed. The electric signal filtered by the low-pass filter 136 is converted into digital signal by the A/D converter 138, and then input into the microcomputer 110.

The electric signal from the infrared detector 108 can be identified by using the control signal for the shaft 125 as a trigger, i.e., it can be identified from which optical filter the infrared light was transmitted through. The electric signal can be identified to which optical filter it corresponds, based on an interval of the output of the control signal for the shaft 125 from the microcomputer to the next output of the control signal for the shaft. By adding the respective electric signals for each of the optical filters and then calculating the average in the memory 112, noise is further reduced. Therefore, such an addition is preferably carried out.

The memory 112 stores correlational data showing correlations between the concentration of a component of a living subject and three electric signals: an electric signal corresponding to the intensity of the first infrared light transmitted through the first optical filter 121, an electric signal corresponding to the intensity of the first infrared light transmitted through the second optical filter 122, and a differential signal of an electric signal corresponding to the intensity of the first infrared light transmitted through the third optical filter 123 and an electric signal corresponding to the intensity of the second infrared light transmitted through the third optical filter 123.

By using the digital signal saved in the memory 112, the microcomputer 110 calculates a digital signal corresponding to the differential signal of the electric signal corresponding to the intensity of the first infrared light transmitted through the third optical filter 123 and the electric signal corresponding to the intensity of the second infrared light transmitted through the third optical filter 123. The microcomputer 110 reads the correlational data stored in the memory 112, and by referring to this correlational data, the digital signal per unit time calculated based on the digital signal stored in the memory 112 is converted to the concentration of a component of a living subject. The memory 112 corresponds to the memory unit of the present invention. The concentration of a component of a living subject converted in the microcomputer 110 is outputted to the display 114 to be displayed.

In this Embodiment, an example is shown by using the shutter 109 as the optical path control unit, but instead of the shutter 109, a shielding plate having an aperture with controllable opening area may be used. The aperture may be set so that when the aperture is half-open, only the first infrared light introduced by the first optical guide path 302 can be transmitted, and when the aperture is complete open, both the first infrared light introduced by the first optical guide path 302 and the second infrared light introduced by the second optical guide path 304 can be transmitted. The electric signal corresponding to the intensity of the second infrared light transmitted through the third optical filter 123 may be obtained by deducting the electric signal corresponding to the intensity of the first infrared light transmitted through the third optical filter 123, from the electric signal corresponding to the intensity of the first infrared light and the second infrared light transmitted through the third optical filter 123.

The first optical filter 121 has spectral characteristics which transmit the infrared light in the wavelength band including the wavelength absorbed by, for example, a component of a living subject to be measured (for example, glucose) (hereinafter, referred to as measurement wavelength band). On the other hand, the second optical filter 122 has spectral characteristics different from the first optical filter 121. The second optical filter 122 has, for example, spectral characteristics which transmit the infrared light in a wavelength band including a wavelength which the measurement target biocomponent does not absorb and which other biocomponent that obstructs the measurement of the target biocomponent absorbs (hereinafter, referred to as reference wavelength band). For such a biocomponent that obstructs the measurement of the target biocomponent, may be selected is a component which is present in a large amount in a living subject other than the measurement target component of a living subject.

For example, glucose shows an infrared absorption spectrum having an absorption peak in the proximity of 9.6 micrometers. Thus, when the measurement target (a component of a living subject) is glucose, the first optical filter 121 preferably has spectral characteristics that transmit the infrared light in the wavelength band including 9.6 micrometers. On the other hand, protein, which is present in a large amount in a living subject, absorbs the infrared light in the proximity of 8.5 micrometers, and glucose does not absorb the infrared light in the proximity of 8.5 micrometer. Thus, the second optical filter 122 preferably has spectral characteristics that transmit the infrared light in the wavelength band including 8.5 micrometers.

The third optical filter 123 has spectral characteristics that transmits the infrared light in the wavelength range that is different from the emissivity of the external ear canal and the emissivity of the eardrum. As is clear from the above Mathematical Expression 5, the emissivity is dependent on the transmittance and the reflectivity. As described above, the reflectivity of a living subject in the infrared range is about 0.03, and the external ear canal and the eardrum show almost the same degree of reflectivity. On the other hand, the transmittance of the external ear canal is in the proximity of 0, since the thickness of the external ear canal is a few centimeters or more. Therefore, in the wavelength range in which the transmittance of eardrums is high, the difference between the emissivity of the external ear canal and the emissivity of eardrum increases.

As is clear from Mathematical Expression 7, the smaller the extinction coefficient of a living subject, that is, the smaller the absorption of light by the living subject, the larger the transmittance. Since about 60 to 70% of a living subject is formed of water, in the wavelength range in which an absorption by water is low, the transmittance of the eardrum becomes high, and the difference between the emissivity of external ear canal and the emissivity of eardrum becomes large. Thus, wavelength characteristics of the third optical filter 123 are set so that at least a portion of the infrared light having a wavelength among 5 to 6 micrometers and 7 to 11 micrometers is transmitted, i.e., the wavelength range which is not greatly absorbed by water.

The correlational data that illustrates correlations between the three electric signals and the concentration of a biocomponent stored in the memory 112 can be obtained, for example, by the steps below. The three electric signals include: (i) the electric signal corresponding to the intensity of the first infrared light that was transmitted through the first optical filter 121; (ii) the electric signal corresponding to the intensity of the first infrared light that was transmitted through the second optical filter 122; and (iii) the differential signal of the electric signal corresponding to the intensity of the first infrared light that was transmitted through the third optical filter 123 and the electric signal corresponding to the intensity of the second infrared light that was transmitted through the third optical filter 123.

First, infrared light irradiated from an eardrum of a patient having a known biocomponent is measured for a concentration (for example, a blood-sugar level). Upon measurement, the following three electric signals are obtained: the electric signal corresponding to the intensity of the first infrared light that was transmitted through the first optical filter 121, the electric signal corresponding to the intensity of the first infrared light that was transmitted through the second optical filter 122, and the differential signal of the electric signal corresponding to the intensity of the first infrared light that was transmitted through the third optical filter 123 and the electric signal corresponding to the intensity of the second infrared light that was transmitted through the third optical filter 123.

Such a measurement for a plurality of patients having different biocomponent concentrations enables obtaining a set of data comprising three electric signals. The three electric signals comprise the electric signal corresponding to the intensity of the first infrared light that was transmitted through the first optical filter 121, the electric signal corresponding to the intensity of the first infrared light that was transmitted through the second optical filter 122, and the differential signal of the electric signal corresponding to the intensity of the first infrared light that was transmitted through the third optical filter 123 and the electric signal corresponding to the intensity of the second infrared light that was transmitted through the third optical filter 123, and the biocomponent concentration corresponding to these electric signals.

Then, correlational data is obtained by analyzing the thus obtained data set. For example, by using multiple regression analysis such as PLS (Partial Least Squares Regression) and neural networks, multivariate analysis is carried out for the following three electric signals and biocomponent concentrations corresponding to these three signals: the electric signal corresponding to the intensity of the first infrared light that was transmitted through the first optical filter 121; the electric signal corresponding to the intensity of the first infrared light that was transmitted through the second optical filter 122; and the differential signal of the electric signal corresponding to the intensity of the first infrared light that was transmitted through the third optical filter 123 and the electric signal corresponding to the intensity of the second infrared light that was transmitted through the third optical filter 123.

By such analysis, a function showing correlations between the following three electric signals and the biocomponent concentrations corresponding to these three signals can be obtained: the electric signal corresponding to the intensity of the first infrared light that was transmitted through the first optical filter 121, the electric signal corresponding to the intensity of the first infrared light that was transmitted through the second optical filter 122, and the differential signal of the electric signal corresponding to the intensity of the first infrared light that was transmitted through the third optical filter 123 and the electric signal corresponding to the intensity of the second infrared light that was transmitted through the third optical filter 123.

Next, by referring to FIG. 1, FIG. 2, and FIG. 3, operation of a bioinformation measurement device in this embodiment is described.

First, upon pressing of a power source switch 101 of a bioinformation measurement device 100 by a user, a power source in a main body 102 is turned on, and the bioinformation measurement device 100 is set to be in a stand-by mode for measurement.

Then, as shown in FIG. 2, a user holds the main body 102 and inserts an insertion portion 104 to an external ear canal 204. Upon insertion, the end of a first light inlet 105 is to be directed toward an eardrum 202. The insertion portion 104 is a conical hollow pipe, with the diameter thereof increasing from the end portion of the insertion portion 104 to the portion thereof connecting with the main body 102. Therefore, the insertion portion 104 is formed so that the insertion portion 104 is not inserted more than the point where the external diameter of the insertion portion 104 equals the internal diameter of the ear cavity 200.

Then, upon pressing of a measurement start switch 103 of the bioinformation measurement device 100 by a user while keeping the bioinformation measurement device 100 at the position where the external diameter of the insertion portion 104 equals the inner diameter of the ear cavity 200 for a microcomputer 110 to start the operation of a chopper 118, a measurement of infrared light irradiated from the eardrum 202 is started.

To the first light inlet 105, infrared light irradiated from the eardrum 202 and the external ear canal 204 enters. On the other hand, since second light inlets 106 are provided at the side faces of the insertion portion 104 so that the second light inlets 106 are not directed toward the eardrum 202 while the insertion portion 104 is inserted in the ear cavity 200, to the second light inlets 106, infrared light irradiated from the external ear canal 204 enters but the infrared light irradiated from the eardrum 202 does not enter.

As is shown in FIG. 2, at the insertion portion 104, the portion between the second light inlets 106 and the first light inlet 105 corresponds to a shielding portion 119 for shielding the second light inlets 106 from the infrared light irradiated from the eardrum 202. Thus, the first infrared light entered from the first light inlet 105 and introduced into the main body 102 through the first optical guide path 302 corresponds to the infrared light irradiated from the eardrum 202 and the external ear canal 204, and the second infrared light entered from the second light inlets 106 and introduced into the main body 102 through the second optical guide path 304 corresponds to the infrared light irradiated from the external ear canal 204.

The microcomputer 110 calculates the differential signal of the electric signal corresponding to the intensity of the first infrared light that was transmitted through the third optical filter 123 and the electric signal corresponding to the intensity of the second infrared light that was transmitted through the third optical filter 123 based on the above analysis. Since the first infrared light corresponds to the infrared light irradiated from the eardrum 202 and the external ear canal 204, and the second infrared light corresponds to the infrared light irradiated from the external ear canal 204, the intensity of this differential signal is an indicator showing the ratio of the infrared light irradiated from the eardrum included in the first infrared light entered from the first light inlet 105.

In the wavelength range that the third optical filter 123 transmits, since the intensity of the infrared light irradiated from the external ear canal 204 is higher than the intensity of the infrared light irradiated from the eardrum 202, when the infrared light irradiated from the external ear canal 204 is included in addition to the infrared light irradiated from the eardrum 202 in the first infrared light, the differential signal mentioned above is in minus value.

The more the infrared light irradiated from the eardrum is included in the first infrared light, the less the intensity of the first infrared light, and therefore the larger the absolute value of the differential signal, the higher the ratio of the infrared light irradiated from the eardrum included in the first infrared light.

The memory 112 stores a pre-set threshold of the differential signal intensity. The microcomputer 110 reads the threshold from the memory 112, and compares the calculated differential signal intensity with the threshold. When the calculated absolute value of the differential signal intensity is smaller than the threshold, the user is notified of an error, by a display on the display 114 with a message that the insertion direction of the insertion portion 104 is misaligned with the eardrum 202, a sound of a buzzer (not shown), or a sound of a speaker (not shown). When an error is notified for not being able to recognize the position of the eardrum 202, the user can shift the bioinformation measurement device 100 to adjust the insertion direction of the insertion portion 104.

The display 114, the buzzer, and the speaker correspond to the warning output unit of the present invention. When the microcomputer 110 determined that the first infrared light includes sufficient infrared light irradiated from the eardrum based on the result of the comparison between the calculated differential signal intensity and the threshold, a timer 156 starts timing.

When the microcomputer 110 determined that a certain period of time passed from the start of the measurement based on the timing signal from the timer 156, the chopper 118 is controlled to shield the infrared light arriving the optical filter wheel 107. Based on this, the measurement ends automatically. At this time, the microcomputer 110 controls the display 114 and the buzzer (not shown), to notify the user the end of the measurement by displaying a message that the measurement ended on the display 114, sounding a buzzer (not shown), or outputting a sound through a speaker (not shown). Since the user can confirm the end of the measurement, the insertion portion 104 is removed out of the ear cavity 200.

The microcomputer 110 reads, from the memory 112, the correlational data showing the correlations between a biocomponent concentration and the electric signal corresponding to the intensity of the first infrared light that was transmitted through the first optical filter 121, the electric signal corresponding to the intensity of the first infrared light that was transmitted through the second optical filter 122, and the electric signal corresponding to the intensity of the first infrared light that was transmitted through the third optical filter 123; and by referring to the correlational data, the electric signal outputted from the A/D converter 138 is converted into the biocomponent concentration. The obtained biocomponent concentration is displayed on the display 114.

As described above, the differential signal of the electric signal corresponding to the intensity of the first infrared light that was transmitted through the third optical filter 123 and the electric signal corresponding to the intensity of the second infrared light that was transmitted through the third optical filter 123 is an index showing the ratio of the infrared light irradiated from the eardrum included in the first infrared light entered from the first light inlet 105. Thus, a correction is carried out with the proportion of the infrared light irradiated from the eardrum included in the first infrared light entered from the first light inlet 105 by using the correlational data including the above differential signal upon obtaining the biocomponent concentration, and the effects of the infrared light irradiated from the external ear canal 204 can be reduced, thereby achieving highly accurate measurement based on the infrared light irradiated from the eardrum 202.

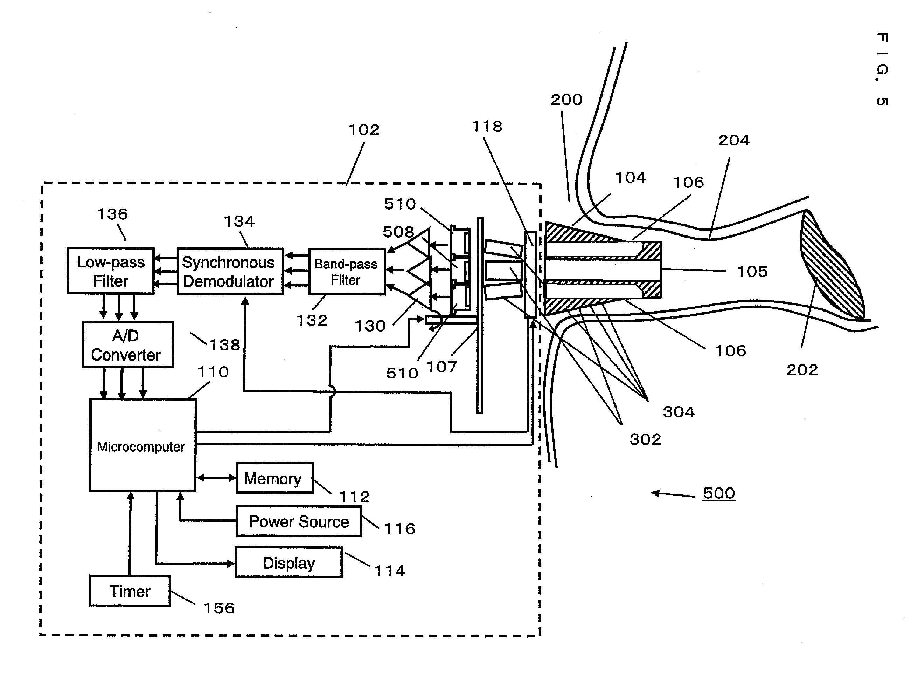

Although an example using a single infrared ray detector 108 is shown in Embodiment 1, the present invention is not limited to this embodiment. A first variation of the bioinformation measurement device of Embodiment 1 is described by referring to FIG. 5. FIG. 5 is a diagram illustrating a configuration of a first variation of the bioinformation measurement device of Embodiment 1. A bioinformation measurement device 500 of a first variation is different from the bioinformation measurement device 100 of Embodiment 1 in that a plurality of infrared ray detectors are used. The same reference numerals are used for the element same as the bioinformation measurement device 100 of Embodiment 1, and descriptions are omitted.

The bioinformation measurement device 500 of the first variation comprises: a first infrared ray detector 508 for detecting first infrared light introduced from the first light inlet 105 through a first optical guide path 302 provided in the insertion portion 104 into the main body 102; and two second infrared ray detectors 510 for detecting second infrared light introduced from the second light inlets 106 into the main body102 through a second optical guide path 304 provided in the insertion portion 104.

The electric signal outputted from the first infrared ray detector 508 and the electric signal outputted from the second infrared ray detector 510 pass through a preliminary amplifier 130, a band-pass filter 132, a synchronous demodulator 134, a low-pass filter 136, and an A/D converter 138, and then in the microcomputer 110, the electric signal outputted from the second infrared ray detector 510 is deducted from the electric signal outputted from the first infrared ray detector 508.

Also, instead of the first infrared ray detector 508 and the two second infrared ray detectors 510, an array type infrared ray detector comprising a first detection region for detecting first infrared light, and two second detection regions for detecting second infrared light may be used.

[Embodiment 2]

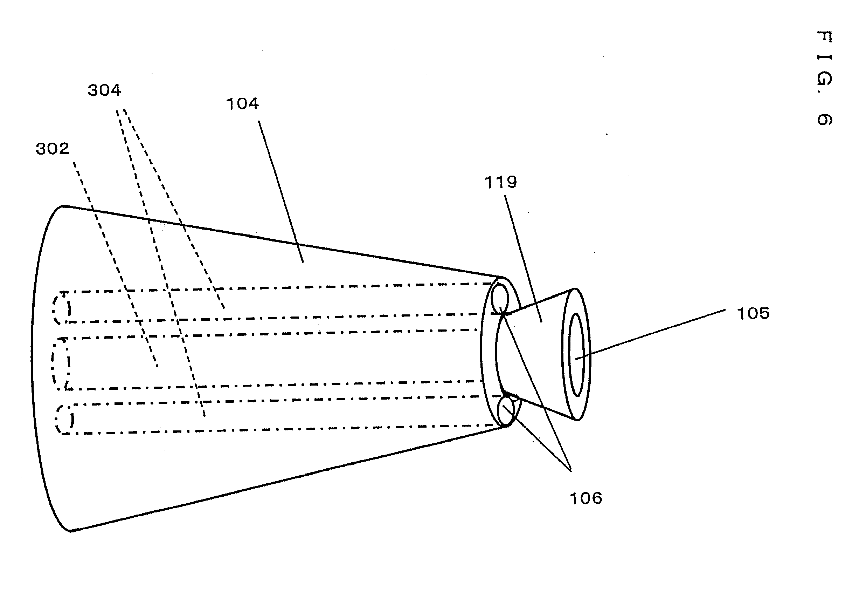

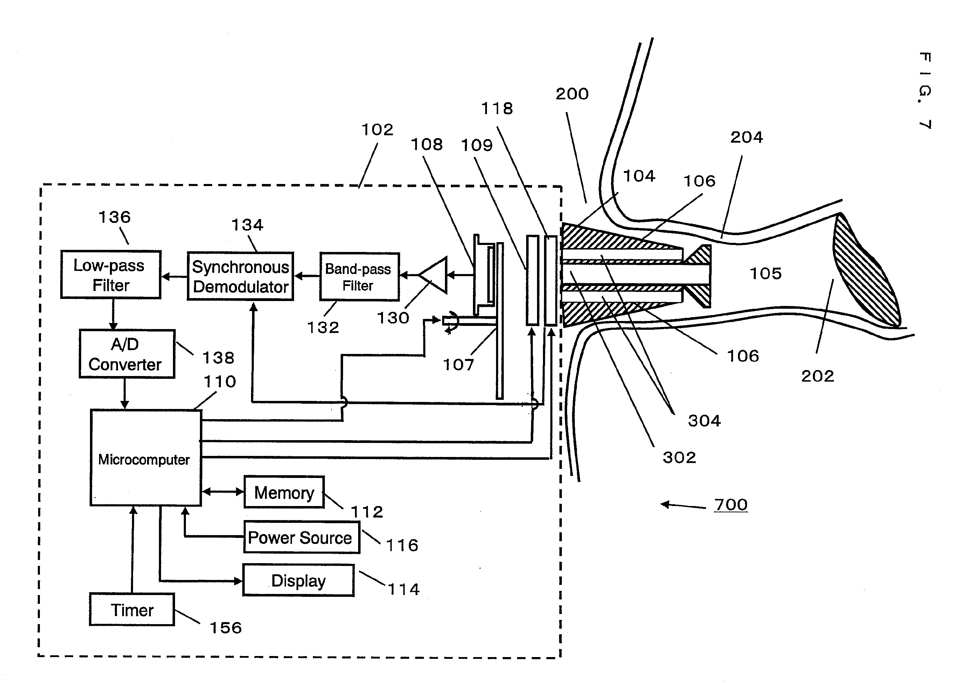

A bioinformation measurement device of Embodiment 2 of the present invention is described by referring to FIGs. 6 and 7. FIG. 6 is a perspective illustration of an insertion portion of a bioinformation measurement device of Embodiment 2 of the present invention, and FIG. 7 shows a configuration of the bioinformation measurement device of Embodiment 2 of the present invention.
The insertion portion 104 of the bioinformation measurement device of this Embodiment includes a shielding portion 119 of truncated cone at the end portion thereof that is directed toward the eardrum 204 when the insertion portion 104 is inserted in the ear cavity 200, and the shielding portion 119 is provided at the end portion of the insertion portion 104 so that the larger bottom face thereof is directed toward the eardrum 202 when the insertion portion 104 is inserted in the ear cavity 200.

At the larger bottom face of the shielding portion 119, a first light inlet 105 is provided, and a first optical guide path 302 communicating with the first light inlet 105 is provided to penetrate the shielding portion 119 and the insertion portion 104 itself. Additionally, second light inlets 106 are provided, at the end portion of the insertion portion 104 that is directed toward the eardrum 202 when the insertion portion 104 is inserted in the ear cavity 200, in the region outside where the shielding portion 119 is provided.

As is clear from FIG. 7, because the shielding portion 119 is positioned at the optical path that links the second light inlets 106 with the eardrum 202 while the insertion portion 104 is being inserted in the ear cavity 200, the shielding portion 119 functions to shield the second light inlets 106 from the infrared light irradiated from the eardrum 202.

The side face of the shielding portion 119 is formed to reflect the infrared light, and while the insertion portion 104 is being inserted in the ear cavity 200, the infrared light irradiated from the external ear canal 204 is reflected at the side face (reflection plane) of the shielding portion 119, to enter the second optical guide path 304 from the second light inlets 106.

The surface of the shielding portion 119 reflects the infrared ray, and therefore preferably is formed of a material with a low degree of infrared ray absorption. Although no particular limitation is made as long as the material reflects the infrared ray, materials such as gold, copper, silver, brass, aluminum, platinum, and iron are preferable. The surface of the shielding portion 119 is preferably smooth, to the extent that it is glossy. The side face (reflection plane) of the shielding portion 119 is preferably tilted, as shown in FIG. 7, with an angle of 45 degrees relative to the second light inlets 106.

Descriptions for other elements of the bioinformation measurement device 700 in this embodiment are omitted, because those are the same with the bioinformation measurement device 100 of Embodiment 1, and the same reference numerals are used. Also, because the operation of the bioinformation measurement device 700 in this embodiment is the same as the bioinformation measurement device 100 in Embodiment 1, descriptions are omitted. With such an arrangement, the effects of the infrared light irradiated from the external ear canal 204 can be decreased as in Embodiment 1, and an accurate measurement based on the infrared light irradiated from the eardrum 202 is made possible.

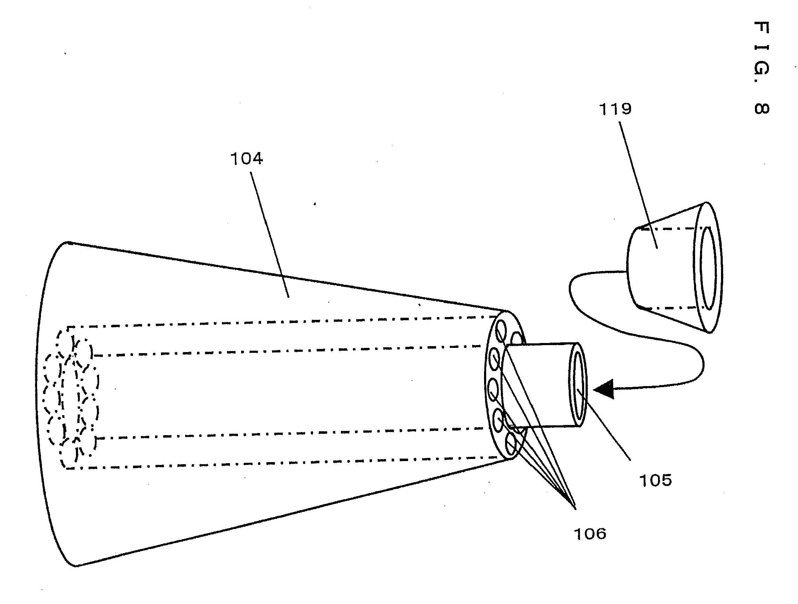

The shielding portion 119 provided in the end portion of the insertion portion 104 of the bioinformation measurement device in this embodiment may be made to be removable from the shielding portion 119, as shown in FIG. 8. FIG. 8 is a perspective illustration of an example of a variation of the insertion portion of the bioinformation measurement device in Embodiment 2 of the present invention. Such an arrangement is preferable in that the shielding portion 119 can be changed in the case when the shielding portion gets dirty by earwax. Additionally, the insertion portion 104 in this variation example includes nine second light inlets 106 and nine second guide paths 304, as shown in FIG. 8.

Although the examples shown in the above embodiment included two second light inlets 106 and two second optical guide paths 304, and nine second light inlets 106 and nine second optical guide paths 304, the number of the second light inlets 106 and the number of the second optical guide paths 304 are not limited these numbers. The second light inlet 106 may be just one, and the second optical guide path 304 may be just one.

Industrial Applicability

The bioinformation measurement device of the present invention is useful in that bioinformation can be measured further accurately.

**Claims**

1. A bioinformation measurement device for measuring bioinformation based on an intensity of infrared light, the bioinformation measurement device comprising:

   an insertion portion to be inserted in an ear cavity;
   a first light inlet and a second light inlet provided at said insertion portion, for introducing infrared light irradiated from said ear cavity into said insertion portion;
   an optical guide path provided in said insertion portion, for guiding first infrared light introduced from said first light inlet and second infrared light introduced from said second light inlet;
   a dispersive element for dispersing said first infrared light and said second infrared light guided by said optical guide path; and
   an infrared ray detector for detecting said first infrared light and said second infrared light dispersed by said dispersive element.

2. The bioinformation measurement device in accordance with Claim 1, further comprising a computing unit for computing bioinformation based on intensities of said first infrared light and said second infrared light detected by said infrared ray detector.

3. The bioinformation measurement device in accordance with Claim 1, wherein said optical guide path comprises:

   a first optical guide path for guiding said first infrared light introduced from said first light inlet; and
   a second optical guide path for guiding said second infrared light introduced from said second light inlet.

4. The bioinformation measurement device in accordance with Claim 1, wherein said second light inlet is configured so that infrared light irradiated from an eardrum is not introduced.

5. The bioinformation measurement device in accordance with Claim 1,
   wherein said insertion portion comprises:

   an end portion directed toward the eardrum upon being inserted into the ear cavity; and
   a side face,
   said first light inlet being provided in said end portion of said insertion portion.

6. The bioinformation measurement device in accordance with Claim 5, wherein said second light inlet is provided at said side face of said insertion portion.

7. The bioinformation measurement device in accordance with Claim 1, further comprising a shielding portion provided at said insertion portion, for shielding said second light inlet from the infrared light irradiated from the eardrum.

8. The bioinformation measurement device in accordance with Claim 1, further comprising an optical path control unit for controlling an optical path of infrared light reaching said infrared ray detector.

9. The bioinformation measurement device in accordance with Claim 1, wherein said computing unit further comprises a warning output unit for comparing the absolute value of the intensity difference between the intensity of said first infrared light and the intensity of said second infrared light with the threshold, and
outputting a warning when said absolute value of said intensity difference is smaller than said threshold.

FIG. 1

FIG. 2

FIG. 3

F I G. 4

FIG. 5

EP 1 955 652 A1

F I G. 7

FIG. 8

**INTERNATIONAL SEARCH REPORT**

| | International application No. |
|---|---|
| | PCT/JP2006/320813 |

A. CLASSIFICATION OF SUBJECT MATTER
*A61B5/1455*(2006.01)i

According to International Patent Classification (IPC) or to both national classification and IPC

B. FIELDS SEARCHED

Minimum documentation searched (classification system followed by classification symbols)
A61B5/1455

Documentation searched other than minimum documentation to the extent that such documents are included in the fields searched
Jitsuyo Shinan Koho          1922-1996    Jitsuyo Shinan Toroku Koho   1996-2006
Kokai Jitsuyo Shinan Koho    1971-2006    Toroku Jitsuyo Shinan Koho   1994-2006

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br>A | JP 7-184866 A  (Casio Computer Co., Ltd.),<br>25 July, 1995 (25.07.95),<br>Par. Nos. [0008] to [0024]; Fig. 1 | 1-6,8<br>7,9 |
| Y<br>A | JP 2002-513604 A  (Optics L.P.),<br>14 May, 2002 (14.05.02),<br>Par. Nos. [0034] to [0048]; Fig. 2 | 1-6<br>7,9 |
| Y | JP 2002-214045 A  (Kabushiki Kaisha Baioekonetto),<br>31 July, 2002 (31.07.02),<br>Par. No. [0014]; Fig. 1 | 1-6 |
| Y | JP 2003-70751 A  (Advanced Medical Kabushiki Kaisha),<br>11 March, 2003 (11.03.03),<br>Par. No. [0011]; Fig. 1 | 1-6 |

☒  Further documents are listed in the continuation of Box C.      ☒   See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>01 November, 2006 (01.11.06) | Date of mailing of the international search report<br>14 November, 2006 (14.11.06) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/320813

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 10-239158 A  (Shimadzu Corp.),<br>11 September, 1998 (11.09.98),<br>Par. Nos. [0011] to [0013]; Fig. 2 | 1-6,8 |
| Y | JP 11-155818 A  (Matsushita Electric Industrial Co., Ltd.),<br>15 June, 1999 (15.06.99),<br>Par. No. [0016]; Fig. 1 | 8 |
| Y | JP 2000-254102 A  (Matsushita Electric Industrial Co., Ltd.),<br>19 September, 2000 (19.09.00),<br>Par. No. [0011]; Fig. 1 | 8 |
| A | JP 2004-329542 A  (Hitachi, Ltd.),<br>25 November, 2004 (25.11.04),<br>Par. Nos. [0015] to [0069]; Figs. 1 to 9 | 1-9 |
| A | JP 2001-503999 A  (Buhato, Yanusu, Emu),<br>27 March, 2001 (27.03.01),<br>Page 15, line 2 to page 19, the last line; Fig. 3 | 1-9 |

Form PCT/ISA/210 (continuation of second sheet) (April 2005)

**INTERNATIONAL SEARCH REPORT**
Information on patent family members

International application No.

PCT/JP2006/320813

| | | | |
|---|---|---|---|
| JP 7-184866 A | 1995.07.25 | JP 3381092 B2 | 2003.02.24 |
| JP 2002-513604 A | 2002.05.14 | AU 744758 B2 | 2002.03.07 |
| | | AU 3968499 A | 1999.11.23 |
| | | CA 2331697 A1 | 1999.11.11 |
| | | EP 1075210 A1 | 2001.02.14 |
| | | US 6002953 A | 1999.12.14 |
| | | WO 1999/56615 A1 | 1999.11.11 |
| JP 2002-214045 A | 2002.07.31 | (Family: none) | |
| JP 2003-70751 A | 2003.03.11 | (Family: none) | |
| JP 10-239158 A | 1998.09.11 | (Family: none) | |
| JP 11-155818 A | 1999.06.15 | (Family: none) | |
| JP 2000-254102 A | 2000.09.19 | (Family: none) | |
| JP 2004-329542 A | 2004.11.25 | CN 1548004 A | 2004.11.24 |
| | | DE 60301868 D1 | 2005.11.17 |
| | | DE 60301868 T2 | 2006.07.27 |
| | | EP 1484006 A1 | 2004.12.08 |
| | | EP 1484006 B1 | 2005.10.12 |
| | | JP 3566276 B1 | 2004.09.15 |
| | | US 2004/225209 A1 | 2004.11.11 |
| | | US 2005/251000 A1 | 2005.11.10 |
| JP 2001-503999 A | 2001.03.27 | AU 711156 B2 | 1999.10.07 |
| | | AU 2599697 A | 1997.12.09 |
| | | EA 2636 B1 | 2002.08.29 |
| | | EP 948284 A1 | 1999.10.13 |
| | | IL 127111 A | 2003.02.12 |
| | | IL 127111 D0 | 1999.09.22 |
| | | JP 3686422 B2 | 2005.08.24 |
| | | PL 184077 B1 | 2002.08.30 |
| | | PL 330044 A1 | 1999.04.26 |
| | | US 5666956 A | 1997.09.16 |
| | | WO 1997/43947 A1 | 1997.11.27 |

Form PCT/ISA/210 (patent family annex) (April 2005)

**EP 1 955 652 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001503999 A